# EUROPEAN PATENT SPECIFICATION

(11) **EP 1 593 736 B1**
(45) Date of publication and mention of the grant of the patent: **08.07.2015**
(21) Application number: 05009824.3
(22) Date of filing: 04.05.2005
(51) Int. Cl.: C12M 1/34, C12Q 1/04

(54) **Microorganisms detecting device and method**
Vorrichtung und Verfahren zum Nachweis von Mikroorganismen
Dispositif et procédé de detection de micro-organismes

(30) Priority: 06.05.2004 JP 2004137319
(43) Date of publication of application: 09.11.2005
(73) Proprietor: Panasonic Healthcare Holdings Co., Ltd., Tokyo 105-8433 (JP)
(72) Inventor: Kono, Eiji, Ora-gun, Gunma 370-0523 (JP); Sekine, Hironobu, Ora-gun, Gunma 370-0523 (JP); Yokoi, Yasuhiko, Hirakata-shi, Osaka 573-0052 (JP); Iwama, Akifumi, Tsukuba-shi, Ibaraki 305-0861 (JP)
(74) Representative: Glawe, Delfs, Moll

(56) References cited:
- US-A- 3 756 920
- US-A- 5 420 017
- US-A- 5 770 395
- PATENT ABSTRACTS OF JAPAN vol. 2003, no. 09, 3 September 2003 (2003-09-03) & JP 2003 144193 A (MITSUBISHI HEAVY IND LTD), 20 May 2003 (2003-05-20)

## Description

### BACKGROUND OF THE INVENTION

The present invention relates to a microorganisms detecting device and a microorganisms detecting method for detecting microorganisms in a sample, and in more detail to a microorganisms detecting device and a microorganisms detecting method to be utilized in diagnosis of infection, tests for microorganisms in food products, detection of microorganisms in circumstances, quality control of cleaning water for semiconductors and the like.

In recent years, with development of the tissue engineering, various techniques associated therewith have been rapidly advanced such as a technique for mitigating the adverse reaction of human bodies to medical appliances by attaching cultured stem cells to surfaces of medical appliances to be introduced into the bodies, and a technique for culturing cells having variety of functions. In such techniques for culturing cells, it is required to prove reliably that cells to be introduced into human bodies have not been contaminated by microorganisms. For this purpose, microorganisms detecting methods have also been developed with the advancement of the tissue engineering. A microorganisms detecting method using precursor phenolic oxidase also is one of such microorganisms detecting methods (refer to, for example, Japanese Patent Application Laid-Open No. 98,798/1997).

However, the method using precursor phenolic oxidase of the prior art suffers a problem from lower sensitivity for detecting microorganisms, particularly gram negative bacteria.

A microorganism detecting device according to the preamble of independent claim 1 is shown from US 3,756,920 A and WO 02/068580 A2.

### SUMMARY OF THE INVENTION

In view of such a problem of the prior art, the present invention provides a microorganisms detecting device and a microorganisms detecting method, which are simple and able to shorten the time for detecting microorganisms and further intend to improve the sensitivity of detection for microorganisms, particularly gram negative bacteria.

Namely, the microorganisms detecting device according to the invention is a device for detecting microorganisms, comprising: a sampling section adapted for taking a sample from a detection target, a sample concentrating section adapted for concentrating the microorganisms existing in the sample taken in the sampling section, said sample concentrating section including filtering means for collecting the microorganisms, a reagent adding section adapted for adding a reagent having a component which changes the reagent color tone by existence of the microorganisms to the sample concentrated in the sample concentrating section, a microorganisms detecting section adapted for detecting the change of the color tone of the reagent added to the sample in the reagent adding section by means of an optical system, a detected result outputting section adapted for outputting the detected result detected in the microorganisms detecting section, and a controller adapted for controlling the sampling section, the sample concentrating section, the reagent adding section, the microorganisms detecting section, and the detected result outputting section, characterized in that the sample concentrating section and reagent adding section comprise a microorganism detecting test tube, the filtering means being provided at the bottom of the microorganism detecting test tube, and a drain tank under the microorganism test tube being connected to a discharging valve and a vacuum pump, wherein the sample concentrating section further includes culturing means by supplying a culture medium to the filtering means via the microorganism detecting test tube, and wherein the reagent adding section supplies the reagent to the filtering means via the microorganism detecting test tube.

According to this invention, it becomes possible to shorten the time for detection with a simple device.

Furthermore, it becomes possible to improve the sensitivity of detection in addition to the above effects.

Moreover, it becomes possible to further improve the sensitivity of detection in addition to the above effects.

Moreover, the microorganism detecting device according to the invention is characterized in that the sample concentrating section includes detecting sensitivity improving means for improving detecting sensitivity for particular microorganisms in the microorganisms detecting section.

According to this invention it becomes possible to improve the sensitivity of detection for particular microorganisms in addition to any effects described above.

Further, the microorganism detecting device according to the invention is characterized in that the detecting sensitivity improving means is breakout of the microorganisms' membrane structures by heating or drying.

According to this invention, it becomes possible to improve the sensitivity of detection for the microorganisms such as gram negative bacteria which permit to improve the sensitivity with their membrane structures being broken in addition to the above effects.

Moreover, the microorganisms detecting device according to the invention is characterized in that the sample concentrating section comprises temperature maintaining means for maintaining the sample at a predetermined temperature.

According to this invention, it becomes possible to concentrate microorganisms to a level sufficient to detect the microorganisms in a short period of time in addition to the above effects, even if the microorganisms contained in a detection target are a very small amount.

Moreover, the microorganisms detecting device according to the invention is characterized in that the optical system comprises a light source outputting light that has wave lengths of the visible light range.

According to this invention, it becomes possible to detect the change in color tone of the reagent with ease in addition to any of above effects.

Moreover, the microorganisms detecting method according to the invention is a method for detecting microorganisms using the device of the invention, comprising: a sampling step for taking a sample from a detection target, a sample concentrating step for concentrating the microorganism existing in the sample taken in the sampling step by means of a filtering means for collecting the microorganisms, a reagent adding step for adding a reagent having a component which changes the reagent color tone by existence of the microorganisms to the sample concentrated in the sample concentrating step, a microorganisms detecting step for detecting the change of the color tone of the reagent added in the reagent adding step, a detected result outputting step for outputting the detected result detected in the microorganisms detecting step, and controlling the sampling step, the sample concentrating step, the reagent adding step, the microorganisms detecting step, and the detected result outputting step by control processing, characterized in that the sample concentration step further includes supplying a culture medium to the filtering means, and the reagent adding step supplies the reagent to the filtering means.

According to this invention, it becomes possible to shorten the time for detection with a simple method.

Moreover, it becomes possible to improve the sensitivity of detection in addition to the above effects.

Furthermore, it becomes possible to further improve the sensitivity of detection in addition to the above effects.

Furthermore, the microorganism detecting method according to the invention characterized in that the sample concentrating step includes detecting sensitivity improving treatment for improving detecting sensitivity for particular microorganisms in the microorganisms detecting step.

According to this invention it becomes possible to improve the sensitivity of detection for particular microorganisms in addition to the effects of any method described above.

Moreover, the microorganism detecting method is characterized in that the detecting sensitivity improving treatment is breakout of the microorganisms' membrane structures by heating or drying.

According to this invention, it becomes possible to improve the sensitivity of detection for the microorganisms such as gram negative bacteria which permit to improve the sensitivity with their membrane structures being broken in addition to the above effects.

That is to say, according to the invention, it is possible to detect the existence of microorganisms securely with the simple detecting device or method, to shorten the time for detection and to improve the sensitivity of detection, particularly sensitivity of detection for microorganisms such gram negative bacteria and the like.

### BRIEF DESCRIPTION OF THE DRAWINGS

FIG. 1 is a conceptual diagram of a microorganisms detecting system;
FIG. 2 is a pattern diagram illustrating the device construction of a sample concentrating section;
FIG. 3 is a graph illustrating a growth curve of microorganisms;
FIG. 4 is a pattern diagram showing a membranes structure of gram negative bacteria;
FIG. 5 is a pattern diagram illustrating the device construction of a microorganisms detecting section;
FIG. 6 is a flow chart illustrating the microorganisms detecting method in Example 1 of the present invention;
FIG. 7 is a flow chart illustrating the microorganisms detecting method in Example 2 of the present invention;
FIG. 8 is a flow chart illustrating the microorganisms detecting method in Example 3 of the present invention; and
FIG. 9 is a flow chart illustrating the microorganisms detecting method in Example 4 of the present invention.

### DETAILED DESCRIPTION OF THE PREFERRED EMBODIMENTS

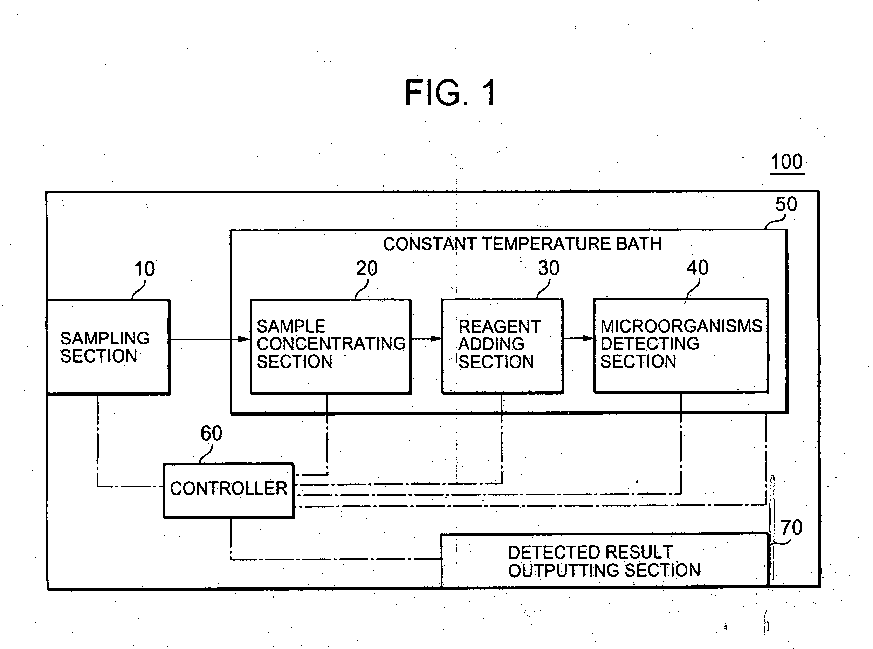

FIG. 1 is a system organization chart of the microorganisms detecting system 100 according to the invention. The microorganisms detecting system 100 comprises a sampling section 10, a sample concentrating section 20, a reagent adding section 30, and a microorganisms detecting section 40, and the sample concentrating section 20, the reagent adding section 30 and the microorganisms detecting section 40 are arranged in a constant temperature bath 50. A controller 60 controls operations of these sections and setting of environmental conditions, and receives detected results from the microorganisms detecting section 40, which are transmitted to a detected result outputting section 70 to output the microorganisms detected results.

In section 10, when exchanging culture medium and the like, a culture medium for culturing cell which may be discarded was added to test tubes. In this case, instead of sampling part of culture medium for detecting as is the case with conventional manners, substantially all the amount of culture medium for culturing cells is employed for detection so that more accurate detected results can be obtained. As the sampling section 10 in the microorganisms detecting system 100 is most in risk of invasion of microorganisms from exterior, it is desired to arrange the microorganisms detecting system 100 in its entirety in a biological clean chamber. Further, it is desired to arrange the sampling section 10 in a biological clean chamber and to employ a structure capable of automatically replacing the culture medium for culturing the cells from the flask for culturing cells into a microorganisms detecting test tube.

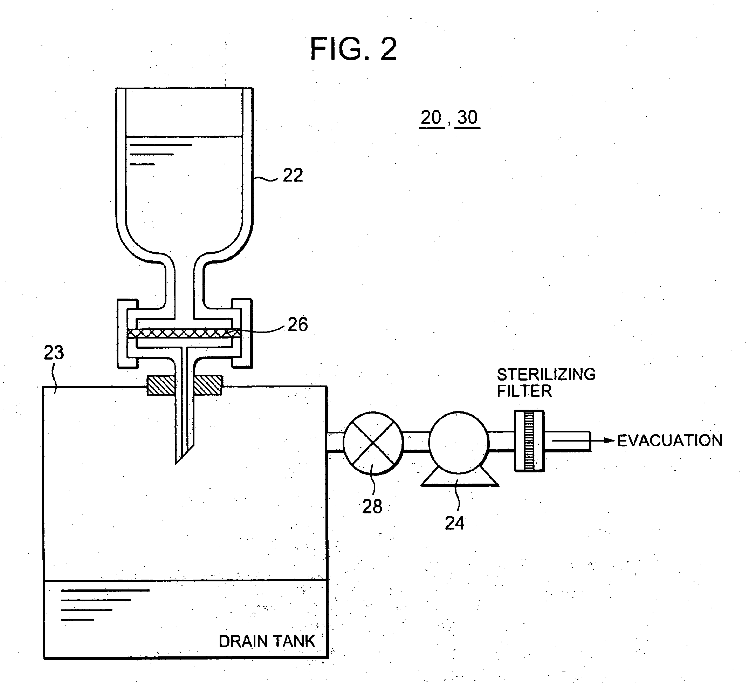

In the sample concentrating section 20, as shown in FIG. 2, in order to improve the sensitivity of detection for microorganisms by increasing and condensing the microorganisms to be detected in the culture medium for culturing cells sampled in the sampling section 10, first a drain tank 23 under a microorganisms detecting test tube 22 is evacuated by a vacuum pump 24 provided on the drain tank 23 to collect the microorganisms in the culture medium by means of a filter 26 at the bottom of the microorganisms detecting test tube 22. The filter 26 has pore size of the order of 0.1 to 10 µm, preferably 0.2 µm in diameter. In this system, a filter of "Ultra Free MC" manufactured by Millipore Co., Ltd. is used.

The culture medium from which microorganisms have been collected is discharged from the microorganisms detecting test tube 22 by the vacuum pump 24, and a discharging valve 28 is closed. Thereafter, a culture medium for culturing the microorganisms is added into the microorganisms detecting test tube 22 from its upper portion. While Glucose-pepton liquid medium (GP liquid medium) is used as a culture medium for culturing microorganisms, other media may be used, such as Soybean-casein-digest liquid medium (SCD liquid medium), Thioglycolic acid medium (TG medium), Sabouraud-glucose medium, Brainheart infusion, Mullerhinton, usual bouillon, or Glucose peptone and the like. Moreover, a plurality of microorganisms detecting test tubes 22 may be previously prepared, and after microorganisms have been collected in the respective test tubes, culture media for culturing plural kinds of microorganisms, which are commensurate with microorganisms expected to be detected, may be added into the microorganisms detecting test tubes, respectively.

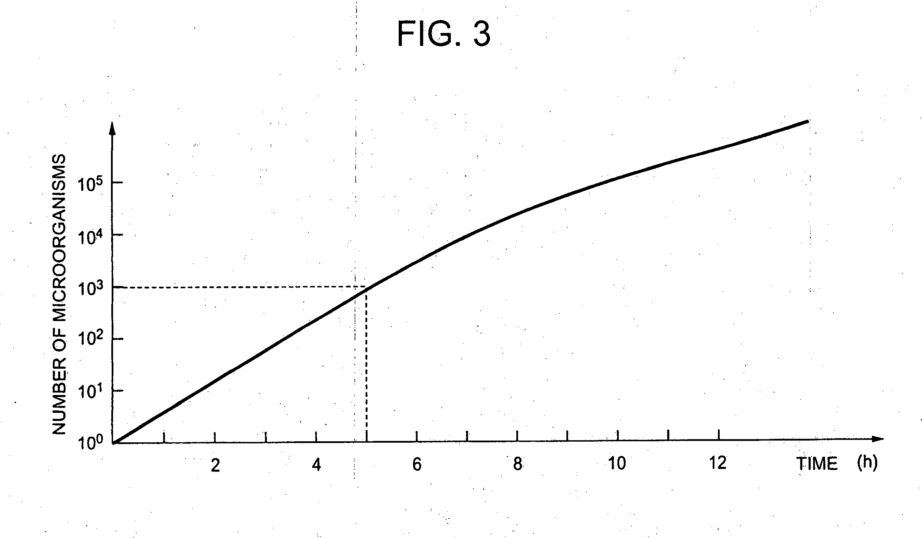

After the culture medium for culturing the microorganisms is added into the microorganisms detecting test tube 22, the microorganisms are cultured at a constant temperature of 30 to 40°C for 3 to 12 hours, preferably 5 to 8 hours. As shown in FIG. 3, the microorganisms have a property increasing twice in number for about 30 minutes although depending on their kinds. Therefore, even if only one microorganism before cultivation, it will breed more than 1000 for five hours.

Microorganisms of fungi such as Candida Albicanis, Candida Lusitaniae, Candida Krusei, Candida Glabrata, Cryptococcus Neoformans, Aspergillus Fumigatus, Pneumocistis Carinii and the like actively grow within a temperature range of about 30°C, while microorganisms of bacteria such as Bacillus Subtilis, Streptococcus Pyogenes, Salmonella Typhimurium, Salmonella Bongori, Salmonella Enteritidis, Escherichia Coli, Staphylococcus Epidermidis, Staphylococcus Aureus, Pseudomonasu Aeruginosa and the like actively grow within a temperature range of about 37°C. Therefore, a plurality of microorganisms detecting test tubes 22 may be previously prepared, and after microorganisms have been collected in the microorganisms detecting test tubes, respectively, the microorganisms may be cultured within temperature ranges of plural kinds commensurate with the microorganisms expected to be detected.

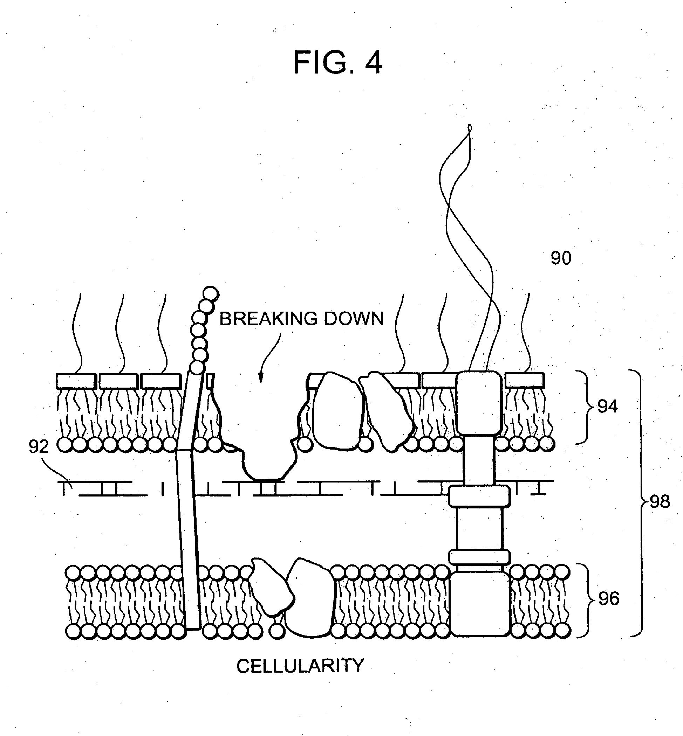

In the case that SLP reagent (reagent A) manufactured by Wako Pure Chemical Industries, Ltd. is used as a reagent, moreover, as the detecting sensitivity of this reagent for gram negative bacteria is lower in comparison with other fungi and bacteria. Therefore, after microorganisms have been cultured in the microorganisms detecting test tube 22, the microorganisms may be broken by the use of drying, heating, ultrasonic wave or surface-active agent. As shown in FIG. 4, the gram negative bacteria 90 have peptide glycan 92 reacting to the reagent A inside an external membrane 94 so that the detecting sensitivity with the reagent A is lower. Therefore, the external membrane 94 or cytoplasmic membrane 96 is broken as described above to cause the peptide glycan 92 which has been between the external membrane 94 and the cytoplasmic membrane 96 of the gram negative bacteria 90 to be exposed outside so that it becomes likely to react to the reagent A.

In the sample concentrating section 20, the microorganisms are cultured to improve the detecting sensitivity for the microorganisms in the manner described above, and the culture medium for culturing the microorganisms is discharged and the treatment for further improving the sensitivity such as breaking the microorganisms is carried out. Thereafter, a reagent is added into the microorganisms detecting test tube 22 in the reagent adding section 30. Although the reagent A is used here, Limulus reagent (reagent B) manufactured by Wako pure Industries and the like may be used. Moreover, a plurality of microorganisms detecting test tubes 22 may be previously prepared, and after microorganisms have been collected in the respective test tubes, reagents of plural kinds may be added into the microorganisms detecting test tubes commensurate with expected microorganisms to be detected, respectively.

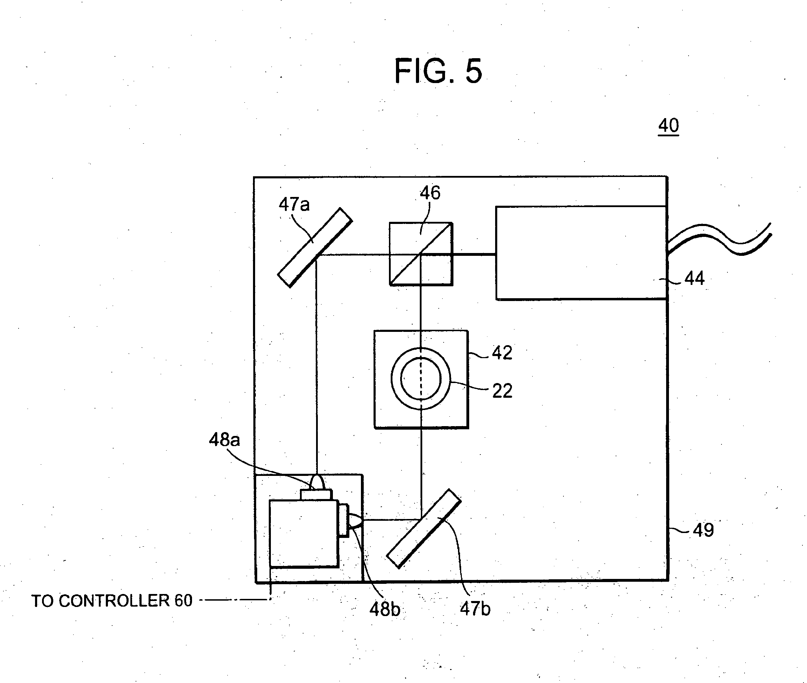

After the reagent A has been added in the reagent adding section 30, existence of microorganisms is optically detected in the microorganisms detecting section 40. As shown in FIG. 5, the microorganisms detecting section 40 comprises a locating stage 42 for the microorganisms detecting test tube 22, a light source (630 nm) 44, a half mirror 46, mirrors 47a and 47b, and light receiving elements 48a and 48b, and these parts are arranged in a light shielding chamber 49. After the reagent A is added into the microorganisms detecting test tube 22, it is required to wait for the progress of reaction for approximately 120 minutes until the color tone in the microorganisms detecting test tube 22 is stabilized. After being stabilized, the microorganisms detecting test tube 22 is located on the locating stage 42 for the microorganisms detecting test tube in the microorganisms detecting section 40. The temperature of the locating stage 42 is set at a temperature suitable for reaction of the reagent, for example, at 30°C with the reagent A or at 37°C with the reagent B. When the location of the microorganisms detecting test tube 22 on the locating stage 42 is completed, light of 630 nm is irradiated from the light source to the test tube to detect the transmitted light at the light receiving elements 48a and 48b. Signal from the light receiving elements 48a and 48b concerning quantity of received light is transmitted to the controller 60. In the controller 60, the quantity of the transmitted light in the light receiving element 48b, that is, the degree of light absorption of the reagent in the microorganisms detecting test tube 22 is calculated with the aid of the signal from the light receiving element 48a as reference signal. These measured results and calculated results are transmitted from the controller 60 to the detected result outputting section 70 from which these results are output as data.

The present invention will then be explained in more detail with reference to examples and the experiment for improving the sensitivity for the gram negative bacteria, although the present invention is not limited to these examples.

### (Example 1)

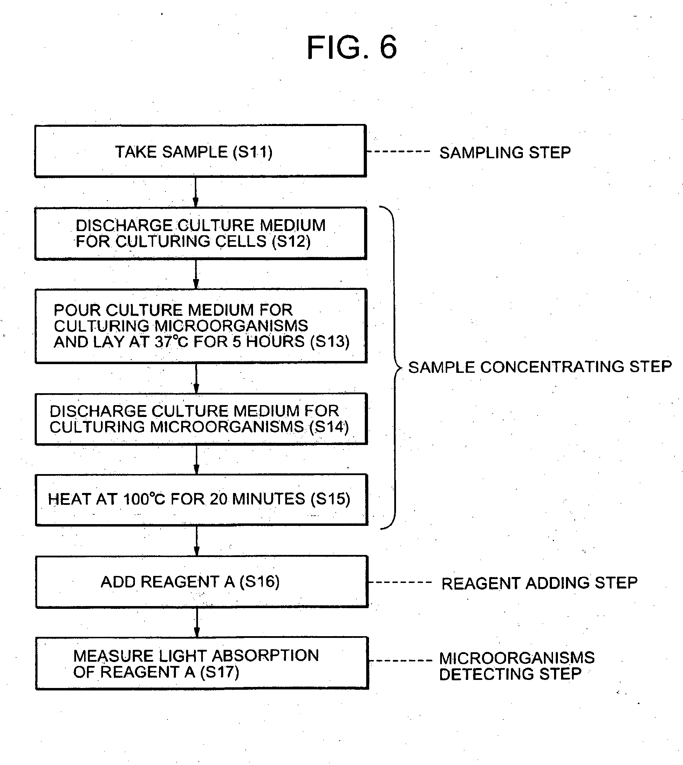

A method for detecting microorganisms in Example 1 with reference to FIG. 6.

Step 1: In the sampling section 20, a sample is taken by replacing a culture medium used for culturing cells from a flask for culturing the cells to a microorganisms detecting test tube 22 (S 11).

Step 2: The discharging valve 28 is opened and the vacuum pump 24 is started so that the culture medium for culturing the cells is discharged through the filter 26 from the bottom of the microorganisms detecting test tube 22 in which the sample has been taken, thereby causing microorganisms to remain on the filter 26 if there have been microorganisms in the culture medium for culturing the cells (S 12)

Step 3: After the discharge of the culture medium for culturing the cells has been completed, the vacuum pump 24 is stopped and the discharging valve 28 is closed and then a culture medium for culturing the microorganisms is poured into the microorganisms detecting test tube 22 from its upper portion. After the pouring of the culture medium for culturing the microorganisms has been completed, the microorganisms detecting test tube 22 is laid to rest in the constant temperature bath 50 at 37°C for 5 hours to culture the microorganisms (S 13).

Step 4: Again the discharging valve 28 is opened and the vacuum pump 24 is started so that the culture medium for culturing the microorganisms is discharged through the filter 26 from the bottom of the microorganisms detecting test tube 22 (S 14).

Step 5: After the discharge of the culture medium for culturing the microorganisms has been completed, the vacuum pump 24 is stopped and the discharging valve 28 is closed. The temperature of the constant temperature bath 50 is set at 100°C and the microorganisms detecting test tube 22 is heated in the bath for 20 minutes, thereby breaking the cell membranes of the microorganisms (S 15).

Step 6: After the heating treatment in the constant temperature bath 50 has been completed, the reagent A is added into the microorganisms detecting test tube 22 from its upper portion (S 16).

Step 7: The microorganisms detecting test tube 22 is arranged on the locating stage 42 in the microorganisms detecting section 40 to measure degree of light absorption or absorbance of the reagent A in the microorganisms detecting test tube 22. In this manner, it is possible to detect whether there have been microorganisms in the culture medium for culturing the cells (S 17).

### (Example 2)

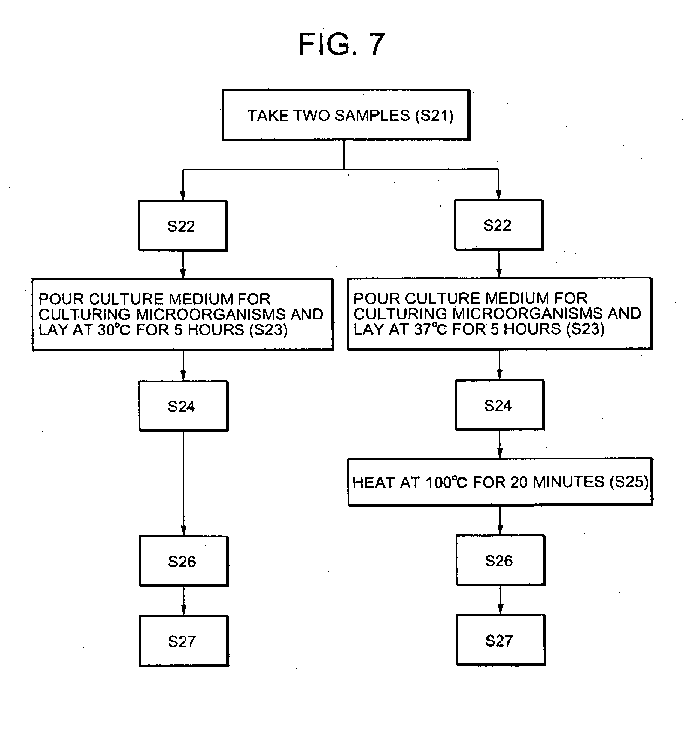

A method for detecting microorganisms in the Example 2 will then be explained with reference to FIG. 7.

Step 1: Two microorganisms detecting test tubes 22 are prepared, and a culture medium used for culturing cells is replaced from the flask for culturing cells to the microorganisms detecting test tubes 22 in the sampling section 20 to prepare two samples (S 21).

Step 2: For each of the two microorganisms detecting test tubes 22, the discharging valve 28 is opened and the vacuum pump 24 is started so that the culture medium for culturing the cells is discharged through the filter 26 from the bottom of the microorganisms detecting test tube accommodating the sample (S 22).

Step 3: After the discharge of the culture medium for culturing the cells has been completed, the vacuum pump 24 is stopped and the discharging valve 28 is closed and then a culture medium for culturing the microorganisms is poured into each of the microorganisms detecting test tubes 22 from its upper portion. After the pouring of the culture medium for culturing the microorganisms has been completed, one microorganisms detecting test tube 22 is laid to rest in the constant temperature bath 50 being set at 30°C for 5 hours to culture the microorganisms. On the other hand, the other microorganisms detecting test tube 22 is also laid to rest in the constant temperature bath 50 being set at 37°C for 5 hours to culture the microorganisms (S 23).

Step 4: For each of the microorganisms detecting test tubes 22, again the discharging valve 28 is opened and the vacuum pump 24 is started so that the culture medium for culturing the microorganisms is discharged through the filter 26 from the bottom of the microorganisms detecting test tube 22 (S 24).

Step 5: After the discharge of the culture medium for culturing the microorganisms has been completed, the vacuum pump 24 is stopped and the discharging valve 28 is closed. The other microorganisms detecting test tube 22 is heated in the constant temperature bath 50 being set at 100°C for 20 minutes. This heating treatment for the one microorganisms detecting test tube 22 is not carried out since it is not necessary for this tube (S 25).

Step 6: After the heating treatment for the other microorganisms detecting tube 22 has been finished (after the step 4 for the one microorganisms detecting test tube 22 has been finished), the reagent A as a reagent is added into the two microorganisms detecting test tubes 22 from their upper portions, respectively (S 26).

Step 7: The two microorganisms detecting test tubes 22 are each arranged on the locating stage 42 of the microorganisms detecting section 40 to measure light absorption or absorbance of the reagent A in the microorganisms detecting test tube 22. In this manner, it is possible to detect whether there have been microorganisms in the culture medium for culturing the cells and further whether there have been fungi or bacteria in the culture medium (S 27).

### (Example 3)

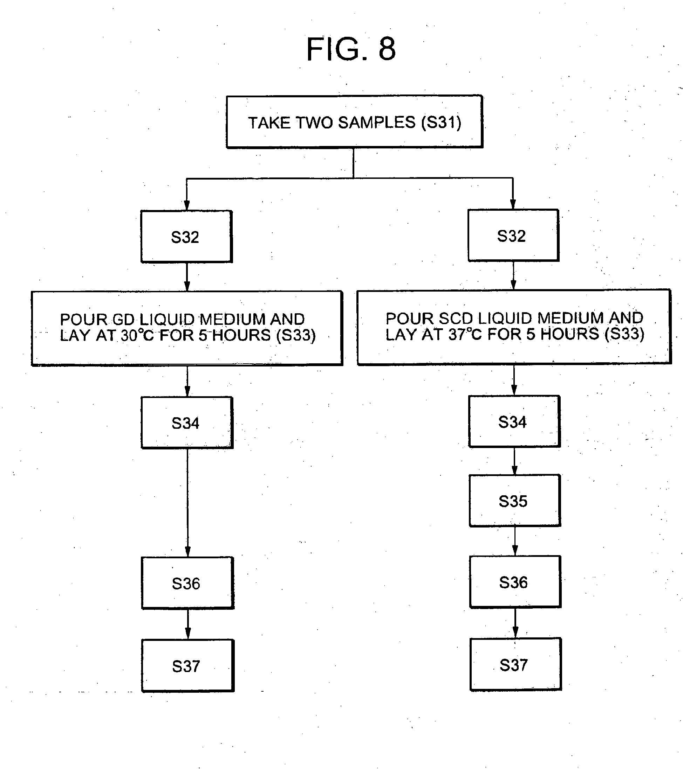

A method for detecting microorganisms in the Example 3 will then be explained with reference to FIG. 8.

Step 1: Two microorganisms detecting test tubes are prepared, and a culture medium used for culturing cells is replaced from the flask for culturing cells to the microorganisms detecting test tubes 22 in the sampling section 20 to prepare two samples (S 31).

Step 2: For each of the two microorganisms detecting test tubes 22, the discharging valve 28 is opened and the vacuum pump 24 is started so that the culture medium for culturing the cells is discharged through the filter 26 from the bottom of the microorganisms detecting test tube accommodating the sample (S 32).

Step 3: After the discharge of the culture medium for culturing the cells has been completed, the vacuum pump 24 is stopped and the discharging valve 28 is closed and then GP liquid medium as a culture medium for culturing the microorganisms is poured into one microorganisms detecting test tube 22 from its upper portion, while SCD liquid medium as a culture medium for culture the microorganisms is poured into the other microorganisms detecting test tube 22 from its upper portion. After the pouring of the culture medium for culturing the microorganisms has been completed, both the microorganisms detecting test tubes 22 may be laid in the constant temperature bath whose temperature is set at 35°C to culture microorganisms. However, the one microorganisms detecting test tube 22 may be laid in the constant temperature bath being set at 30°C and the other test tube 22 may be laid in the constant temperature bath set at 37°C, respectively for 5 hours to culture the microorganisms, thereby enabling the measurement with higher sensitivity (S 33).

Step 4: For each of the microorganisms detecting test tubes 22, again the discharging valve 28 is opened and the vacuum pump 24 is started so that the culture medium for culturing the microorganisms is discharged through the filter 26 from the bottom of the microorganisms detecting test tube 22 (S 34).

Step 5: After the discharge of the culture medium for culturing the microorganisms has been completed, the vacuum pump 24 is stopped and the discharging valve 28 is closed. The other microorganisms detecting test tube 22 is heated in the constant temperature bath 50 being set at 100°C for 20 minutes. This heating treatment for the one microorganisms detecting test tube 22 is not carried out since it is not necessary for this tube (S 35).

Step 6: After the heating treatment for the other microorganisms detecting tube 22 has been finished (after the step 4 for the one microorganisms detecting test tube 22 has been finished), the reagent A as a reagent is added into the two microorganisms detecting test tubes 22 from their upper portions, respectively (S 36).

Step 7: The two microorganisms detecting test tubes 22 are each arranged on the locating stage 42 of the microorganisms detecting section 40 to measure light absorption or absorbance of the reagent A in the microorganisms detecting test tube 22. In this manner, it is possible to detect whether there have been microorganisms in the culture medium for culturing the cells and further whether there have been fungi or bacteria in the culture medium (S 37).

### (Example 4)

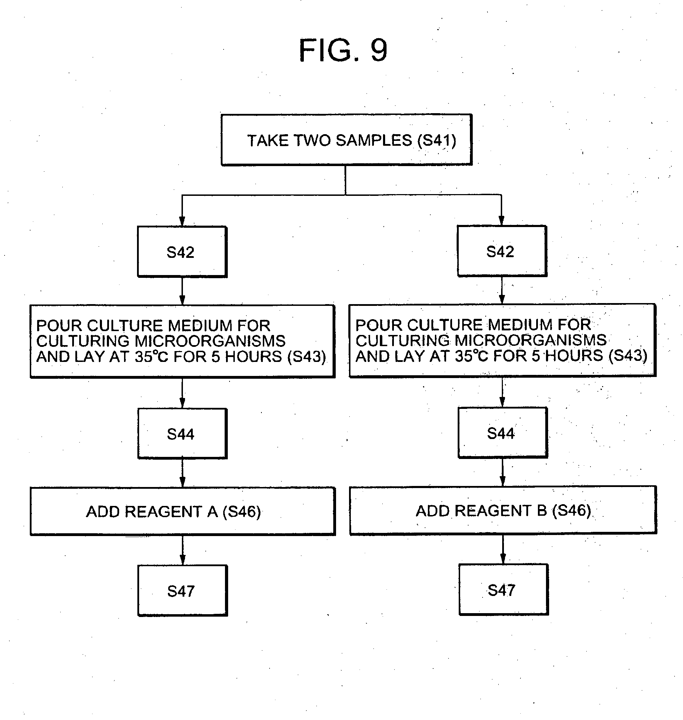

Finally, a method for detecting microorganisms in the Example 4 will then be explained with reference to FIG. 9.

Step 1: Two microorganisms detecting test tubes are prepared, and a culture medium used for culturing cells is replaced from the flask for culturing cells to the microorganisms detecting test tubes 22 in the sampling section 20 to prepare two samples (S 41).

Step 2: For each of the two microorganisms detecting test tubes 22, the discharging valve 28 is opened and the vacuum pump 24 is started so that the culture medium for culturing the cells is discharged through the filter 26 from the bottom of the microorganisms detecting test tube accommodating the sample (S 42).

Step 3: After the discharge of the culture medium for culturing the cells has been completed, the vacuum pump 24 is stopped and the discharging valve 28 is closed and then a culture medium for culturing the microorganisms is poured into each of the microorganisms detecting test tubes 22 from its upper portion. After the pouring of the culture medium for culturing the microorganisms has been completed, both the microorganisms detecting test tubes 22 are laid to rest in the constant temperature bath 50 being set at 35°C for 5 hours to culture the microorganisms (S 43).

Step 4: For each of the microorganisms detecting test tubes 22, again the discharging valve 28 is opened and the vacuum pump 24 is started so that the culture medium for culturing the microorganisms is discharged through the filter 26 from the bottom of the microorganisms detecting test tube 22 (S 44).

Step 5: After the discharge of the culture medium for culturing the microorganisms has been completed, the vacuum pump 24 is stopped and the discharging valve 28 is closed. The reagent A is added into the one microorganisms detecting test tube 22 from its upper portion, and the reagent B is added into the other microorganisms detecting test tube 22 from its upper portion (S 46).

Step 6: The two microorganisms detecting test tubes 22 are each arranged on the locating stage 42 of the microorganisms detecting section 40 to measure light absorption or absorbance of the respective reagent in the microorganisms detecting test tube 22. In this manner, it is possible to detect whether there have been microorganisms in the culture medium for culturing the cells without treatment for breaking the cell membranes as by heating (S 47).

### <Experiment for improving sensitivity for gram negative bacteria>

The experiment was executed by the use of *Escherichia Coli* (IF03301) as a sample of the gram negative bacteria, *Bacillus Subtilis* (IF03035) as a sample of the gram positive bacteria and *Aspergillus Nigger* (IF06341) as a sample of the fungi.

First, the samples were prepared by making serial 1:10 dilution of the respective microorganism, and the samples were each separately attached with 50 µl to three micro plates (only one micro plate for the fungi). Then, these samples were divided into three groups, that is, the first group in that without being particularly treated, the samples are mixed with the (untreated) reagent A, the second group in that after being heat treated, the samples are mixed with the reagent A, and the third group in that after being treated by drying, the samples are mixed with the reagent A. After being mixed with the reagent A, using the micro-plate reader (sunrise thermo) manufactured by TECAN Co., Ltd., variances in 630 nm absorbance were measured with an interval of 30 seconds, and time of variances was measured using values of absorbance variance of 10 mOD as a threshold.

The number of bacteria (viable bacteria) of each dilute group was determined with the culture on the plates of the respective dilute group and analytical curves obtained therefrom. In the heat treated group, after the samples were heated at 100°C for 20 minutes and added with suitable amount of water for adjustment, the reagent A was added to them. In the treated group by vacuum drying, after the samples were heated at 40°C for 2 hours and added with water for adjustment, the regent A was added to them.

Results of the experiment is shown in Table 1.

**Table 1**

| Number of bacteria | Gram negative bacteria | | | Gram positive bacteria | | | Fungi |
|---|---|---|---|---|---|---|---|
| | Untreated | Heated | Dried | Untreated | Heated | Dried | Untreated |
| 10⁵ /ml | × | ○ | ○ | ○ | ○ | ○ | ○ |
| 10⁵ /ml | × | ○ | ○ | ○ | ○ | ○ | ○ |
| 10⁵ /ml | × | ○ | ○ | ○ | ○ | ○ | ○ |
| 10⁵ /ml | × | × | × | × | × | × | × |
| 10⁵ /ml | × | × | × | × | × | × | × |

As shown in Table 1, in the gram positive bacteria there is no difference in sensitivity of detection between untreated, heated and dried groups, while in the gram negative bacteria the sensitivity of detection of the heated or dried group is improved to the level substantially equal to those in the gram positive bacteria and fungi even with the number of bacteria which could not be detected with the untreated gram negative bacteria.

Accordingly, it becomes apparent that the affirmation of existence of microorganisms is possible only with one kind of reagent A by the heating or drying treatment for breaking cell membranes.

### <Other items>

While the method for detecting microorganisms contained in the culture medium for culturing cells has been explained in the Examples, the detection target containing microorganisms are not limited to the culture medium for culturing cells and may include washing water to be used in producing steps for foods, and liquid foods and beverages such as juice, wine and the like, air in the sanitarily managed circumstances (filters through which the air flows) or cleaning water for semiconductors.

Although the filtering device of end flow type is used for collecting microorganisms in the sample concentrating section 20, a flat membrane may be used for collecting microorganisms. By attaching porous electrodes made of carbon to the flat membrane and causing electric current to flow through the electrodes, more effective collection of microorganisms becomes possible. Other than such a method using a filter, microorganisms can be collected by centrifugal separation, adsorption using beads, and the like.

While the GP liquid medium is used as a culture medium for culturing microorganisms, SCD liquid medium, TG medium, Sabouraud glucose medium, Brainheart infusion, Mullerhinton, usual bouillon, or glucose peptone may be used. In the case using the culture medium such as GP liquid medium, SCD liquid medium, TG medium, Mullerhinton and the like, after the culture medium for culturing microorganisms has been discharged, purified water is poured into a microorganisms detecting test tube so that the step of cleaning the microorganisms detecting test tube is inserted before adding a reagent, whereby more accurate detection becomes possible.

Some examples of the fungi and bacteria are cited in the above description. However, detection is sufficiently possible using other than these such as fungi belonging to the genus Candida, genus Hansenula, genus Saccharomyces, genus Trichosporon, genus Cryptococcus, genus Aspergillus, genus Penicillium, genus Blastomyces, genus Coccidioides, genus Pneumocystis, genus Malassezia, genus Debaryomyces and the like, and bacteria belonging to the genus Bacillus, genus Streptococcus, genus Pseudomonas, genus Escherichia, genus Staphylococcus, genus Klebsiella, genus Serratia, genus Shigella, genus Vibrio, genus Campylobacter, genus Clostridium, genus Yersinia and like.

The microorganisms detecting section is the device for measuring the absorbance of the reagents using the light source of 630 nm in the above explanation. However, the wave length is not limited to 630 nm insofar as the wave length is in a marginal zone capable of measuring variances in color tone of reagents. Although the absorbance is measured in the above explanation, fluorescent intensity or reflected light may be measured.

The present invention relates to a general purpose microorganisms detecting device and method capable not only of detecting microorganisms contained in a culture medium for culturing cells but also detecting microorganisms in a short period of time with higher sensitivity from all kinds of fluids (liquids and gases) from which detection of microorganisms is desired, such as washing water to be used in producing steps for foods, and liquid food and beverages such as juice, wine and the like, air in the sanitarily managed circumstances (filters through which the air flows) or cleaning water for semiconductors.

## Claims

1. A microorganism detecting device comprising:
a sampling section (10) adapted for taking a sample from a detection target,
a sample concentrating section (20) adapted for concentrating the microorganisms existing in the sample taken in the sampling section, said sample concentrating section including filtering means (26) for collecting the microorganisms,
a reagent adding section (30) adapted for adding a reagent having a component which changes the reagent color tone by existence of the microorganisms to the sample concentrated in the sample concentrating section,
a microorganisms detecting section (40) adapted for detecting the change of the color tone of the reagent added to the sample in the reagent adding section by means of an optical system,
a detected result outputting section (70) adapted for outputting the detected result detected in the microorganisms detecting section, and
a controller (70) adapted for controlling the sampling section, the sample concentrating section, the reagent adding section, the microorganisms detecting section, and the detected result outputting section,
**characterized in that**
the sample concentrating section (20) and reagent adding section (30) comprise a microorganism detecting test tube (22), the filtering means (26) being provided at the bottom of the microorganism detecting test tube (22), and a drain tank (23) under the microorganism test tube (22) being connected to a discharging valve (28) and a vacuum pump (24),
wherein the sample concentrating section (20) further includes culturing means by supplying a culture medium to the filtering means (26) via the microorganism detecting test tube (22), and
wherein the reagent adding section (30) supplies the reagent to the filtering means (26) via the microorganism detecting test tube (22).

2. The microorganism detecting device as set forth in claim 1, **characterized in that** the sample concentrating section includes detecting sensitivity improving means for improving detecting sensitivity for particular microorganisms in the microorganisms detecting section.

3. The microorganism detecting device as set forth in claim 2, **characterized in that** the detecting sensitivity improving means is breakout of the microorganisms' membrane structures by heating or drying.

4. The microorganism detecting device as set forth in any one of claims 1 - 3, **characterized in that** the sample concentrating section comprises temperature maintaining means for maintaining the sample at a predetermined temperature.

5. The microorganism detecting device as set forth in any one of claims 1 - 4, **characterized in that** the optical system comprises a light source outputting light that has wave lengths of the visible light range.

6. A microorganism detecting method using the device according to claim 1 comprising:
a sampling step for taking a sample from a detection target,
a sample concentrating step for concentrating the microorganisms existing in the sample taken in the sampling step by means of a filtering means for collecting the microorganisms,
a reagent adding step for adding a reagent having a component which changes the reagent color tone by existence of the microorganisms to the sample concentrated in the sample concentrating step,
a microorganisms detecting step for detecting the change of the color tone of the reagent added in the reagent adding step,
a detected result outputting step for outputting the detected result detected in the microorganisms detecting step, and
controlling the sampling step, the sample concentrating step, the reagent adding step, the microorganisms detecting step, and the detected result outputting step by control processing,
**characterized in that**
the sample concentration step further includes supplying a culture medium to the filtering means, and the reagent adding step supplies the reagent to the filtering means.

7. The microorganism detecting method as set forth claim 6, **characterized in that** the sample concentrating step includes detecting sensitivity improving treatment for improving detecting sensitivity for particular microorganisms in the microorganisms detecting step.

8. The microorganism detecting method as set forth in claim 7, **characterized in that** the detecting sensitivity improving treatment is breakout of the microorganisms' membrane structures by heating or drying.

## Patentansprüche

1. Mikroorganismus-Detektorvorrichtung mit:
einem Probenabschnitt (10), der zum Nehmen einer Probe von einem Detektionsziel ausgebildet ist,
einem Probenkonzentrations-Abschnitt (20), der zum Konzentrieren der Mikroorganismen, die in der in dem Probenabschnitt genommenen Probe enthalten sind, ausgebildet ist, wobei der Probenkonzentrations-Abschnitt Filtermittel (26) zum Sammeln der Mikroorganismen aufweist,
einem Reagensadditions-Abschnitt (30), der zum Zufügen eines Reagens mit einer Komponente ausgebildet ist, die den Reagens-Farbton durch die Existenz der Mikroorganismen in der Probe, die in dem Probenkonzentration-Abschnitt konzentriert wurde, ändert
einem Mikroorganismus-Detektionsabschnitt (40), der zur Erfassung der Änderung des Farbtons des Reagens geeignet ist, das der Probe in dem Reagens-Additions-Abschnitt zugefügt wurde, mittels eines optischen Systems,
einem Detektorergebnis-Ausgabeabschnitt (70), der zum Ausgeben des erfassten Ergebnisses, das in dem Mikroorganismen-Detektionsabschnitten detektiert wurde, ausgebildet ist, und
einem Controller (70), der zum Steuern des Probenabschnittes, des Probenkonzentration-Abschnittes, des Reagensadditions-Abschnittes, des Mikroorganismen-Detektionsabschnittes und des Detektionsergebnis-Ausgabeabschnittes ausgebildet ist,
**dadurch gekennzeichnet, dass**
der Probenkonzentrations-Abschnitt (20) und der Reagensadditions-Abschnitt (30) ein Mikroorganismendetektions-Testrohr (22) aufweist, wobei die Filtermittel (26) am Boden des Mikroorganismendetektions-Testrohrs (22) vorgesehen sind und wobei ein Ablauftank (23) unter dem Mikroorganismendetektions-Testrohr (22) mit einem Ablassventil (28) und einer Vakuumpumpe (24) verbunden ist,
wobei der Probenkonzentrations-Abschnitt (20) ferner Kultivierungsmittel durch Zuführen eines Kulturmediums an die Filtermittel (26) über das Mikroorganismendetektions-Testrohr (22) aufweist, und
wobei der Reagensadditions-Abschnitt (30) das Reagens an die Filtermittel (26) über das Mikroorganismendetektions-Testrohr (22) zuführt.

2. Mikroorganismus-Detektionsvorrichtung nach Anspruch 1, **dadurch gekennzeichnet, dass** der Probenkonzentrations-Abschnitt Detektionsempfindlichkeits-Verbesserungsmittel aufweist zum Verbessern der Detektionsempfindlichkeit für bestimmte Mikroorganismen in dem Mikroorganismendetektions-Abschnitt.

3. Mikroorganismen-Detektionsvorrichtung nach Anspruch 2, **dadurch gekennzeichnet, dass** die Detektionsempfindlichkeits-Verbesserungsmittel ein Aufbrechen der Membranstrukturen der Mikroorganismen durch Aufheizen oder Trocknen sind.

4. Mikroorganismus-Detektionsvorrichtung nach einem der Ansprüche 1-3, **dadurch gekennzeichnet, dass** der Probenkonzentrations-Abschnitt ein Temperatur-Haltemittel zum Halten der Probe bei einer vorgegebenen Temperatur aufweist.

5. Mikroorganismus-Detektionsvorrichtung nach einem der Ansprüche 1-4, **dadurch gekennzeichnet, dass** das optische System eine Lichtquelle aufweist, die Licht mit der Wellenlänge des sichtbaren Lichtbereichs ausgibt.

6. Mikroorganismus-Detektionsverfahren unter Verwendung der Vorrichtung nach Anspruch 1 mit:
einem Probenschritt zum Nehmen einer Probe von einem Detektionsziel,
einem Probenkonzentrations-Schritt zum Konzentrieren der Mikroorganismen, die in der Probe enthalten sind, die in dem Probenschritt genommen wurden, mittels einer Filtereinrichtung zum Sammeln der Mikroorganismen,
einem Reagensadditions-Schritt zum Zufügen eines Reagens mit einer Komponente, die den Reagens-Farbton durch Existenz der Mikroorganismen der Probe, die in dem Probenkonzentration-Schritt konzentriert wurde, ändert,
einem Mikroorganismen-Detektionsschritt zum Erfassen der Änderung des Farbtons des Reagens, das in dem Reagensadditions-Schritt zugefügt wurde,
einem Detektorergebnis-Ausgabe-Schritt zum Ausgaben des Detektorergebnisses, das in dem Mikroorganismus-Detektionsschritt detektiert wurde, und
Steuern des Probenschrittes, des Probenkonzentrations-Schrittes, des Reagensadditions-Schrittes, des Mikroorganismen-Detektorschrittes und des Detektionsergebnis-Ausgabeschrittes durch Steuerverarbeitung,
**dadurch gekennzeichnet, dass**
der Probenkonzentrations-Schritt ferner die Zufuhr eines Kulturmediums an die Filtermittel aufweist, wobei der Reagensadditions-Schritt das Reagens an die Filtermittel zuführt.

7. Mikroorganismus-Detektionsverfahren nach Anspruch 6, **dadurch gekennzeichnet, dass** der Proben-Konzentrationsschritt eine Detektionsempfindlichkeits-Verbesserungsbehandlung umfasst zum Verbessern der Detektionsempfindlichkeit für bestimmte Mikroorganismen in dem Mikroorganismen-Detektionsschritt.

8. Mikroorganismus-Detektionsverfahren nach Anspruch 7, **dadurch gekennzeichnet, dass** die Detektionsempfindlichkeits-Verbesserungsbehandlung ein Aufbrechen der Membranstruktur der Mikroorganismen durch Aufheizen oder Trocknen ist.

## Revendications

1. Dispositif de détection de micro-organismes comprenant :
une section d'échantillonnage (10) conçue pour prélever un échantillon à partir d'une cible de détection,
une section de concentration d'échantillon (20) conçue pour concentrer les micro-organismes existant dans l'échantillon prélevé dans la section d'échantillonnage, ladite section de concentration d'échantillon comprenant des moyens de filtration (26) pour collecter les micro-organismes,
une section d'ajout de réactif (30) conçue pour ajouter un réactif ayant un composant qui change la teinte du réactif par l'existence des micro-organismes à l'échantillon concentré dans la section de concentration d'échantillon,
une section de détection de micro-organismes (40) conçue pour détecter le changement de la teinte du réactif ajouté à l'échantillon dans la section d'ajout de réactif au moyen d'un système optique,
une section de sortie de résultat détecté (70) conçue pour délivrer le résultat détecté qui a été détecté dans la section de détection de micro-organismes, et
un contrôleur (70) conçu pour commander la section d'échantillonnage, la section de concentration d'échantillon, la section d'ajout de réactif, la section de détection de micro-organismes et la section de sortie de résultat détecté,
**caractérisé en ce que**
la section de concentration d'échantillon (20) et la section d'ajout de réactif (30) comprennent un tube à essais de détection de micro-organismes (22), les moyens de filtration (26) étant prévus dans le fond du tube à essais de détection de micro-organismes (22), et un réservoir de drainage (23) sous le tube à essais de micro-organismes (22) étant relié à une vanne de décharge (28) et à une pompe à vide (24),
dans lequel la section de concentration d'échantillon (20) comprend en outre des moyens de culture en fournissant un milieu de culture aux moyens de filtration (26) par l'intermédiaire du tube à essais de détection de micro-organismes (22), et
dans lequel la section d'ajout de réactif (30) fournit le réactif aux moyens de filtration (26) par l'intermédiaire du tube à essais de détection de micro-organismes (22).

2. Dispositif de détection de micro-organismes selon la revendication 1, **caractérisé en ce que** la section de concentration d'échantillon comprend des moyens d'amélioration de sensibilité de détection pour améliorer la sensibilité de détection pour des micro-organismes particuliers dans la section de détection de micro-organismes.

3. Dispositif de détection de micro-organismes selon la revendication 2, **caractérisé en ce que** les moyens d'amélioration de sensibilité de détection consistent en la rupture des structures de membrane des micro-organismes par chauffage ou séchage.

4. Dispositif de détection de micro-organismes selon l'une quelconque des revendications 1 à 3, **caractérisé en ce que** la section de concentration d'échantillon comprend des moyens de maintien de température pour maintenir l'échantillon à une température prédéterminée.

5. Dispositif de détection de micro-organismes selon l'une quelconque des revendications 1 à 4, **caractérisé en ce que** le système optique comprend une source de lumière délivrant une lumière qui a des longueurs d'onde dans la plage de la lumière visible.

6. Procédé de détection de micro-organismes en utilisant le dispositif selon la revendication 1 comprenant :
une étape d'échantillonnage pour prélever un échantillon à partir d'une cible de détection,
une étape de concentration d'échantillon pour concentrer les micro-organismes existant dans l'échantillon prélevé à l'étape d'échantillonnage à l'aide de moyens de filtration pour collecter les micro-organismes,
une étape d'ajout de réactif pour ajouter un réactif ayant un composant qui change la teinte du réactif par l'existence des micro-organismes à l'échantillon concentré à l'étape de concentration d'échantillon,
une étape de détection de micro-organismes pour détecter le changement de la teinte du réactif ajouté à l'étape d'ajout de réactif,
une étape de sortie de résultat détecté pour sortir le résultat détecté qui a été détecté à l'étape de détection de micro-organismes, et
commander l'étape d'échantillonnage, l'étape de concentration d'échantillon, l'étape d'ajout de réactif, l'étape de détection de micro-organismes et l'étape de sortie de résultat détecté par un traitement de commande,
**caractérisé en ce que**
l'étape de concentration d'échantillon comprend en outre la fourniture d'un milieu de culture aux moyens de filtration, et l'étape d'ajout de réactif fournit le réactif aux moyens de filtration.

7. Procédé de détection de micro-organismes selon la revendication 6, **caractérisé en ce que** l'étape de concentration d'échantillon comprend un traitement d'amélioration de sensibilité de détection pour améliorer la sensibilité de détection pour des micro-organismes particuliers à l'étape de détection de micro-organismes.

8. Procédé de détection de micro-organismes selon la revendication 7, **caractérisé en ce que** le traitement d'amélioration de sensibilité de détection consiste en la rupture des structures de membrane des micro-organismes par chauffage ou séchage.
